# EUROPÄISCHE PATENTSCHRIFT

(11) **EP 0 307 904 B1**
(45) Veröffentlichungstag und Bekanntmachung des Hinweises auf die Patenterteilung: **10.02.1993**
(21) Anmeldenummer: 88115114.6
(22) Anmeldetag: 15.09.1988
(51) Int. Cl.: A61K 9/22

(54) **Umhüllte Retardformen**
Covered sustained-release forms
Formes à liberation prolongée enrobées

(30) Priorität: 18.09.1987 CH 3621/87
(43) Veröffentlichungstag der Anmeldung: 22.03.1989
(73) Patentinhaber: CIBA-GEIGY AG, 4002 Basel (CH)
(72) Erfinder: Sinnreich, Joel, Dr., CH-4059 Basel (CH)
(74) Vertreter: Zumstein, Fritz, Dr.

(56) Entgegenhaltungen:
- EP-A- 0 121 331
- EP-A- 0 147 780
- CH-A- 647 161
- US-A- 4 207 890

## Beschreibung

Gegenstand der vorliegenden Erfindung ist eine umhüllte Retardform sowie ein Verfahren zur Herstellung dieser Retardform.

Mehrere Wirkstoffe, welche in üblichen Darreichungsformen wie Tabletten oder Kapseln verabreicht und im Magen wegen des rasch erfolgenden Zerfalls dieser Darreichungsformen innerhalb kurzer Zeit freigesetzt werden, weisen nach dem Transport ihrer Hauptmenge in resorptionsfähige Abschnitte des Gastrointestinaltrakts, insbesondere im Duodenum und anschliessenden Bereichen des Dünndarms, ein unbefriedigendes Resorptionsverhalten auf. So besteht bei wasserlöslichen Wirkstoffen im Duodenum die Gefahr einer schnellen Aufnahme von zu grossen Mengen mit entsprechenden Nebenwirkungen. Bei Wirkstoffen mit schlechter Wasserlöslichkeit werden nur geringe Mengen resorbiert und ungelöste Mengen in weniger gut resorbierende Abschnitte des Gastrointestinaltrakts weitertransportiert. Daher haben orale Darreichungsformen mit verzögerter, kontinuierlicher und kontrollierter Abgabeleistung im Bereich des Magens verschiedene Vorteile:
1. Die Anzahl der Verabreichungen lässt sich generell herabsetzen.
2. Effektive Wirkstoffkonzentrationen lassen sich auf gleichmässig hohem therapeutischen Niveau lange aufrechterhalten, so dass eventuell auftretende unerwünschte Nebenwirkungen durch überhöhte Initialdosis zu Beginn der Verabreichung vermindert werden und der therapeutische Effekt mit erhöhter Wahrscheinlichkeit eintritt.

In der Schweizerischen Patentschrift 647 161 ist ein tablettenförmiges mehrschichtiges Abgabesystem für Wirkstoffe beschrieben, das über eine starre äussere Hülle und einen expandierenden Kern verfügt. Die Abgabe des Wirkstoffs erfolgt durch Diffusion durch die Hülle nach Expansion des Kerns durch Treibmittel oder osmotischen Druck.

In der Europäischen Patentanmeldung 121 331 sind elastische, beutelförmige Behälter für Wirkstoffe beschrieben, welche bei Kontakt mit Magensaft durch Quellung eines Hydrogels expandieren können und den Wirkstoff gezielt im Magensaft freisetzen.

In der U.S. Patentschrift 3,901,232 werden Kapseln beschrieben, welche nach ihrer Auflösung im Magen eine Abgabevorrichtung für den Wirkstoff freisetzen. Diese Abgabevorrichtung ist mit einem ballonförmigen Auftriebskörper verbunden, der ein bei Körpertemperatur verdampfbares Treibmittel enthält, z.B. Diäthyläther, Methylformiat, Neopentan etc.. Durch Verdampfen des Treibmittels wird der Auftriebskörper mit Gas gefüllt, schwimmt auf dem Mageninhalt und verhindert, dass die mit dem Auftriebskörper befestigte Abgabevorrichtung den Magen beim üblichen Entlehrungsvorgang via Pylorus verlässt. Der Wirkstoff kann beispielsweise aus dem matrixähnlichen Material der Abgabevorrichtung kontrolliert abgegeben werden.

Diese Darreichungsform ist bereits aufgrund der verwendeten Treibmittel nachteilig, welche für pharmazeutische Zwecke ungeeignet bzw. sogar toxisch sind, z.B. Methylformiat. Ausserdem ist aufgrund der komplizierten Anordnung der Bestandteile die Herstellung technisch aufwendig.

In der Deutschen Offenlegungsschrift (DE-A) 3 527 852 werden fetthaltige pharmazeutische Zusammensetzungen beschrieben, welche als spezifisch leichtere Phase auf dem Magensaft schwimmen und den Wirkstoff verlangsamt abgeben.

Bekanntlich dämpfen Fette nach ihrer Aufnahme im Magen die Peristaltik, so dass durch den Anteil an Fetten in dieser Formulierung, insbesondere gesättigten Fetten, die periodische Entleerung des Magens verzögert, weniger Mageninhalt weitertransportiert und damit die Resorptionszeit etwas verlängert wird. Allerdings wird bei jeder grösseren Nahrungsaufnahme die zusammenhängende, schwimmfähige, fetthaltige Phase so zerkleinert, dass der Pylorus das Verlassen grosser Mengen dieser zerkleinerten Phase nicht mehr verhindern kann. Die Resorption im Duodenum bleibt aufgrund des rasch erfolgenden Weitertransports ungenügend.

Der vorliegenden Erfindung liegt die Aufgabe zugrunde, eine verbesserte Darreichungsform mit verzögerter und kontrollierter Abgabe herzustellen. Bei oraler Anwendung soll die Darreichungsform trotz periodischer Entleerungsvorgänge länger als 4 Stunden, bevorzugt länger als 24 Stunden, im Magen verbleiben und selbst bei nachfolgender Nahrungsaufnahme weiterhin eine kontinuierliche Abgabe gewährleisten. Die erfindungsgemässe Retardform ist gekennzeichnet durch
a) eine bei Kontakt mit Magensaft expandierende Komponente bestehend aus einem Kohlendioxid-abgabefähigen Stoff und einem pharmazeutischen Wirkstoff,
b) eine im Magen expansionsfähige und für Magensaft permeable, die Komponente a) umgebende Umhüllung aus Polyvinylalkohol in Form eines Sachets, gegebenenfalls vermischt mit Weichmachern, und gegebenenfalls
c) eine die Komponente a) und die Membran b) umgebende Umhüllung, welche durch Einwirken von Magensaft nach der Einnahme zerfällt.

Die weiter vorn und im folgenden verwendeten Begriffe und allgemeinen Definitionen haben im Rahmen der Beschreibung der vorliegenden Erfindung vorzugsweise die folgenden Bedeutungen:
Der Begriff Retardform bezeichnet Darreichungsformen, welche im Vergleich mit konventionellen Darreichungsformen wie gewöhnlichen Tabletten oder Kapseln den Wirkstoff verzögert unter Vermeidung einer unerwünscht überhöhten Initialdosis, kontinuierlich über einen längeren Zeitraum und kontrolliert auf therapeutisch wirksamem Niveau abgeben.

Generell haben Retardformen viele Vorteile, die in der Literatur beschrieben sind, siehe R. Voigt, Lehrbuch der Pharmazeutischen Chemie, Verlag Chemie Weinheim, SS. 679 ff.. So lassen sich z.B. Nebenwirkungen besser vermeiden und die therapeutische Breite erhöhen. Ausserdem wird der Wirkstoff besser ausgenutzt, so dass man die zu applizierende Dosis und/oder die Zahl der Verabreichungen herabsetzen kann. Retardformen sind für verschiedene Applikationsarten bekannt geworden, z.B. transdermal, intramuskulär oder oral.

Die erfindungsgemässe Retardform kann als therapeutisches System mit wertvollen pharmakologischen Eigenschaften in der Human- und Veterinärmedizin nicht nur therapeutisch sondern auch prophylaktisch auf dem für den jeweiligen Wirkstoff vorgesehenen Indikationsgebiet mit der vorgeschriebenen Höchstdosierung angewendet werden. Die erfindungsgemässe Retardform eignet sich für die orale Applikation.

Die bei Kontakt mit Magensaft expandierende Komponente a) der erfindungsgemässen Retardform enthält einen Stoff, welcher z.B. nach oraler Verabreichung der Retardform durch Einwirken von Magensaft, bzw. der darin enthaltenen Wasserstoffionen, das Treibmittel Kohlendioxid entwickelt. Die expandierende Umhüllung b), welche die Komponente a) umgibt, ist als wasserdurchlässiges, aber mehr oder weniger Gas-undurchlässiges Sachet geformt, welches die Komponente a) mit dem das Treibmittel entwickelnden Stoff, z.B. Natriumhydrogencarbonat, und dem Wirkstoff enthält. Durch die Entwicklung des Treibmittels bläht sich dieses Sachet auf und nimmt bis zu 24 Stunden ein vergrössertes Volumen ein. Dieser gasgefüllte "Beutel" ist auf der wässrigen Phase schwimmfähig und wird dabei vom Pylorus zurückgehalten. Während der Verweilzeit im Magen wird der in der Komponente a) enthaltene Wirkstoff durch die Membran des Sachets langsam an die umgebende Körperflüssigkeit vorzugsweise durch Diffusion abgegeben. Da Magensaft stets weitertransportiert wird, gelangt in das Duodenum kontinuierlich und über einen längeren Zeitraum hinweg Wirkstoff, so dass dieser dort verlängert resorbiert wird. Die erfindungsgemässe Retardform gewährleistet daher eine kontinuierliche Wirkstoffabgabe verbunden mit gleichmässiger Resorption.

Die bei Kontakt mit Magensaft expandierende Komponente a) bewirkt eine Volumenvergrösserung der expansionsfähigen Membran b). Die Volumenvergrösserung erfolgt durch Verwendung von geeigneten Treibmitteln.

Geeignete, Treibmittel entwickelnde Stoffe, sind z.B. Feststoffe, welche z.B. durch Einwirken von Magensaft bzw. der darin enthaltenen Wasserstoffionen das Kohlendioxid freisetzen. Solche Treibmittel entwickelnden Stoffe sind zur Abgabe von Kohlendioxid befähigt, z.B. pharmazeutisch verwendbare ein- und zweibasische Salze der Kohlensäure, z.B. Alkalimetallhydrogen- oder Alkalimetallcarbonate oder Erdalkalimetallcarbonate.

Solche ein- und zweibasischen Salze der Kohlensäure sind insbesondere Natriumhydrogen- oder Natriumcarbonat, Kaliumcarbonat, Calciumcarbonat, Magnesiumcarbonat oder Mischungen davon. Den genannten Carbonaten kann zur Verstärkung der CO₂-Entwicklung die in Brauseformulierungen (effervescent mixtures) übliche Säurekomponente, z.B. Natriumdihydrogen- oder Dinatriumhydrogenphosphat, Natriumtartrat, Natriumascorbat oder Natriumcitrat, zugesetzt sein. Ferner eignen sich Hefen, die ebenfalls zur Entwicklung von Kohlendioxidgas fähig sind. Bei Verwendung von Hefen, z.B. Bäckerhefe, setzt man der Formulierung die erforderlichen Nährstoffe, z.B. Glucose, hinzu.

Ein in der expandierenden Komponente a) vorhandener pharmazeutischer Wirkstoff ist in wässriger Phase, z.B. Magensaft, leicht löslich bzw. in gelöstem Zustand resorbierbar. In wässriger Phase mässig oder schwerlösliche Wirkstoffe sind in der Komponente a) vorzugsweise als wasserlösliche, pharmazeutisch annehmbare Salze vorhanden, z.B. als Hydrobromid, Hydrochlorid, Mesylat, Acetat, Succinat, Lactat, Tartrat, Fumarat, Sulfat, Maleat, etc.

Geeignete pharmazeutische Wirkstoffe sind z.B. entzündungshemmende Mittel, z.B. Indomethacin, Acetylsalicylsäure, Ketoprofen, Ibuprofen, Mefenaminsäure, Dexamethason, Natriumdexamethasonsulfat, Hydrocortison oder Prednisolen, Coronardilatatoren, z.B. Nifedipin, Isosorbitdinitrat, Nitroglycerin, Dilthiazem, Trapidil, Dipyridamol oder Dilazep, Prostaglandine, z.B. Prostaglandin E₁, E₂ oder F_{2α}, periphere Vasodilatatoren, z.B. Ifenprodil, Cinepazetmaleat, Cyclandelat, Cinnarizin oder Pentoxyphyllin, Antibiotica, z.B. Ampicillin, Amoxycillin, Cephalexin, Cefradin, Cefaclor, Erythromycin, Bacampicillin, Minocyclin oder Chloramphenicol, Antiseptica für die Harnwege, z.B. Pipemidinsäure oder Nalixidinsäure, geschwürhemmende Mittel, z.B. Sulperid, Cetraxat oder Gefarnat, fiebersenkende Mittel, z.B. Phenacitin, Isopropylantipyrin, Acetaminophen oder Benzydamin, krampflösende Mittel, z.B. Propanthelin, Atropin oder Scopolamin, Antitussiva und Antiasthmatica, z.B. Theophyllin, Aminophyllin, Methylephedrin, Procatechol, Trimethoquinol, Codein, Clofedanolol oder Dextromethorphan, Diuretica, z.B. Furosemid oder Acetazolamid, muskelentspannende Mittel, z.B. Chlorphenesincarbamat, Tolperison, Eperison oder Baclofen, schwache Beruhigungsmittel, z.B. Oxazolam, Diazepam, Clotiazepam, Medazepam, Temazepam oder Fludiazepam, starke Beruhigungsmittel, z.B. Sulpirid, Clocapramin oder Zotepin, Beta-Blocker, z.B. Pindolol, Propranolol, Carteolol, Metoprolol oder Labetalol, Antiarrhythmica, z.B. Procainamid, Disopyramid, Ajimalin oder Quinidin, Antigichtmittel wie Allopurinol, Anticoagulanzien wie Ticlopidin, Antiepileptica, z.B. Phenytoin, Valproat oder Carbamazepin, Antihistaminica, z.B. Chlorpheniramin, Clemastin, Mequitazin, Alimemazin, Cyproheptadin, Mittel gegen Uebelkeit und Schwindel, z.B. Diphenidol, Methochlopromid, Domperidon oder Bethahistin, Blutdrucksenkende Mittel, z.B. Reserpin, Rescinnamin, Methyldopa, Prazosin, Clonidin oder Budralazin, Sympathomimetica, z.B. Dihydroergotamin, Isoproterenol oder Etilefrin, Expectoranzien, z.B. Bromhexin, Corbocistein, L-Ethylcystein oder L-Methylcystein, orale Antidiabetica, z.B. Glibencamid oder Tolbutamid, kardiovaskuläre Mittel, z.B. Ubidecarenon oder Adenosin, Antazida, z.B. Natriumhydrogen- oder Natriumcarbonat, Kaliumcarbonat oder Calciumcarbonat oder Rehydrationssalze, z.B. Kaliumchlorid.

Die Komponente a) kann ausserdem die üblichen und zur Herstellung von oralen Darreichungsformen wie Tabletten, Pellets, Mikrokapseln oder Retardsystemen wie Matrixsystemen oder oralen osmotischen Systemen zur Zeit verwendbaren pharmazeutischen Formulierungshilfsstoffe enthalten, z.B. grenzflächenaktive Stoffe, z.B. sogenannte Tenside, z.B. anionische Tenside vom Typ Alkylsulfat, z.B. Natrium-, Kalium- oder Magnesium-n-dodecylsulfat, -n-tetradecyl-sulfat, -n-hexadecyl-sulfat oder -n-octadecyl-sulfat, Alkyläthersulfat, z.B. Natrium-, Kalium- oder Magnesium-n-dodecyloxyäthylsulfat, -n-tetradecyloxyäthylsulfat, -n-hexadecyl-oxyäthylsulfat oder -n-octadecyloxyäthylsulfat oder Alkansulfonat, z.B. Natrium-, Kalium- oder Magnesium-n-dodecansulfonat, -n-tetradecansulfonat, -n-hexadecansulfonat oder -n-octadecansulfonat.

Geeignete Tenside sind ferner nichtionische Tenside vom Typ Fettsäure-Polyhydroxyalkoholester wie Sorbitanmonolaurat, -oleat, -stearat oder -palmitat, Sorbitantristearat oder -trioleat, Polyoxyäthylen-Addukte von Fettsäure-Polyhydroxyalkoholestern wie Polyoxyäthylen-sorbitanmonolaurat, -oleat, -stearat, -palmitat, -tristearat oder -trioleat, Polyäthylenglycol-Fettsäureester wie Polyoxyäthylenstearat, Polyäthylenglycol-400-stearat oder Polyäthylenglycol-2000-stearat, insbesondere Aethylenoxid-Propylenoxid Blockcopolymere vom Typ Pluronics® (BWC) oder Synperonic® (ICI), Myristinsäure und deren Kondensationsprodukte oder Aethylenoxidhomopolymere mit einem Polymerisationsgrad von ca. 2'000 bis 100'000, welche z.B. unter dem Handelsnamen Polyox® (Union Carbide) bekannt geworden sind.

Weitere Hilfsstoffe sind die bei der Herstellung von Tabletten, Pellets, Mikrokapseln, Granulaten, Matrixsystemen, oralen osmotischen Systemen (OROS) verwendeten üblichen Hilfsstoffe, z.B. Bindemittel, Gleitmittel, Fliessmittel, Dispergiermittel, Füllmittel etc.. So können übliche Hilfsstoffe wie Gelatine, Lactose, Saccharose, Sorbit, Mannit oder Cellulose, insbesondere mikrokristalline Cellulose, oder Magnesiumstearat zusätzlich zu den genannten Hilfsstoffen verwendet werden.

Die hydrophile Membran b), welche am Anwendungsort expansionsfähig und für Körperflüssigkeit permeabel ist, besteht aus einem plastischen oder wachsartigen, pharmazeutisch verwendbaren polymeren Material, welches für das Treibmittel nur wenig oder überhaupt nicht gasdurchlässig ist. Aufgrund seiner hydrophilen Eigenschaften kann es Körperflüssigkeit wie Magensaft aufnehmen und kontrollierte Mengen des physiologisch wirksamen Stoffs verlangsamt und kontinuierlich mittels Diffusion oder gegebenenfalls Anwendung von Osmose abgeben.

Geeignet ist Polyvinylalkohol mit einem Hydrolysegrad höher als 92 % (vollverseifter Polyvinylalkohol), insbesondere höher als 97 %, z.B. MOWIOL der 98-er Reihe, z.B. MOWIOL 4-98, 10-98, 20-98, 28-99, 56-98 und 66-100.

Diesen Materialien können weitere Hilfsstoffe, z.B. Weichmacher, welche die Elastizität der Umhüllung verbessern, z.B. Glycerin, Polyäthylenglycol-Fettsäureester wie Polyäthylenglycol-400-stearat oder Polyäthylenglycol-2000-stearat, Triäthylcitrat, Diäthylphthalat, Diäthylsebacat etc. zugesetzt sein. Die Menge an zugesetztem Weichmacher beträgt ca. 0,01-60 Gew.%, bezogen auf die Gesamtmenge des therapeutischen Systems.

Die Anordnung der Komponenten a) und der Membran b) kann verschiedenartig erfolgen. In einer bevorzugten Ausführungsform stellt die Komponente a) den Kern der Retardform dar, welcher bei Kontakt mit Magensaft expandiert. Dieser Kern kann aus einem Treibmittel entwickelnden Stoff wie Natriumhydrogencarbonat und einem pharmazeutischen Wirkstoff bestehen. Ist der Treibmittel entwickelnde Stoff selbst physiologisch wirksam, z.B. als Antacidum wie Natriumhydrogencarbonat, kann der Kern ausschliesslich daraus bestehen. Dabei ist die expansionsfähige, permeable Membran b) als Umhüllung des Kerns angeordnet.

Der Kern kann auch statt von einer von mehreren Umhüllungen aus expansionsfähigem, permeablem Material umgeben sein. Bei einer solchen mehrschichtigen Anordnung kann sich zwischen den einzelnen Schichten ebenfalls eine Formulierung des physiologisch wirksamen Stoffs befinden bzw. Bestandteile der Formulierung, z.B. Treibmittel wie Natriumhydrogencarbonat. Bei einer mehrschichtigen Anordnung kann man eine noch längere Verweildauer der Darreichungsform am Wirkungsort, z.B. im Magen, erzielen. Ausserdem können in der expansionsfähigen, permeablen Membran b) selbst physiologisch wirksame Stoffe enthalten sein.

Die erfindungsgemässe Retardform kann mit einer Umhüllung c) versehen sein, welche die Komponente a) und die Membran b) umgibt und durch Einwirken von Körperflüssigkeit am Anwendungsort ohne Verzögerung zerfällt und aus einem Filmüberzug oder vorzugsweise einer kapselförmigen Umhüllung besteht.

Geeignete Filmüberzüge verzögern die Wirkstoffabgabe nur gering oder überhaupt nicht. Bevorzugt sind wasserlösliche Filmüberzüge mit einer Stärke von ca. 20 µm bis ca. 100 µm.

Als Filmüberzugsmaterialien eignen sich besonders hydrophile Cellulosederivate, wie Celluloseäther, z.B. Methylcellulose, Hydroxypropylcellulose oder besonders Hydroxypropylmethylcellulose, Gemische von Polyvinylpyrrolidon oder eines Copolymerisates von Polyvinylpyrrolidon und Polyvinylacetat mit Hydroxypropylmethylcellulose, Gemische von Schellack mit Hydroxypropylmethylcellulose, Polyvinylacetat oder dessen Copolymeren mit Polyvinylpyrrolidon, oder Gemische von wasserlöslichen Cellulosederivaten, wie Hydroxypropylmethylcellulose, und wasserunlöslicher Aethylcellulose. Diese eigentlichen Ueberzugsmittel können, wenn erwünscht, im Gemisch mit anderen Hilfsstoffen, wie Talk, Netzmitteln, z.B. Polysorbaten (z.B. zur Erleichterung des Auftrags), oder Pigmenten (z.B. zur Kennzeichnung) verwendet werden. Diese Ueberzüge werden, je nach Löslichkeit der Komponenten, in wässriger Lösung oder in organischer Lösung (z.B. Lösungen von Schellack oder Aethylcellulose in organischen Lösungsmitteln) aufgetragen. Ferner können auch Gemische von an sich wasserunlöslichen Acrylaten, z.B. das Copolymerisat von Acrylsäureäthylester und Methacrylsäuremethylester, die in wässriger Dispersion verwendet werden, mit wasserlöslichen Hilfsstoffen, z.B. Lactose, Polyvinylpyrrolidon, Polyäthylenglykol oder Hydroxypropylmethylcellulose, verwendet werden.

Statt mit einem filmartigen Ueberzug können die erfindungsgemässen Retardformen mit einer kapselförmigen Umhüllung versehen werden. Bevorzugt sind Hartgelatinekapseln mit hoher Wasserlöslichleit und/oder Quellfähigkeit. Bevorzugt sind Steckkapseln der Grösse 0.

Die erfindungsgemässe Retardform kann unterschiedlich geformt sein und beispielsweise rund, oval, oblong, rohrförmig u.ä. sein sowie verschiedene Grössen in Abhängigkeit von der Füllmenge haben. Das therapeutische System kann ausserdem transparent, farblos oder gefärbt sein, um diesem Produkt ein individuelles Aussehen bzw. sofortige Erkennbarkeit zu verleihen.

Die erfindungsgemässe umhüllte Retardform lässt sich nach bekannten Verfahren herstellen, indem man z.B. die Komponente a) aus einem bei Kontakt mit Körperflüssigkeit expandierenden Kern mit dem pharmazeutischen Wirkstoff herstellt, z.B. indem man einen zur Kohlendioxid-Entwicklung befähigten Stoff wie Natriumhydrogencarbonat mit einem Wirkstoff oder einer Wirkstoffkombination vermischt, granuliert oder komprimiert, diesen Kern der Komponente a) mit der expansionsfähigen, die Komponente a) umgebenden Membran b) in Form einer Umhüllung umgibt und die so umhüllte Formuliermasse gegebenenfalls mit der a) und b) umgebenden Umhüllung c) versieht, welche schnell bei Kontakt mit Wasser zerfällt.

Dies kann z.B. durch Verpacken der aus der Komponente a) und der Membran b) bestehenden Formulierungsmasse in Steckkapseln geeigneter Grösse erfolgen.

In einer bevorzugten Ausführungsform des Verfahrens stellt man die expansionsfähige, permeable, die Komponente a) umgebende Membran b) zuerst her, indem man z.B. ein homogenes Gemisch von Polyvinylalkohol und Zusätzen wie Weichmachern, z.B. Glycerin und/oder Polyäthylenglycol-400-stearat, durch Auflösen in gegebenenfalls erwärmtem Wasser und Verdampfen zu Schichten geeigneter Stärke, z.B. 100 µm, herstellt bzw. eine Lösung von Polyvinylalkohol in Wasser (ohne Zusätze) verdampfen lässt. Die Schichten schneidet man in Streifen geeigneter Grösse und trägt die aus der Komponente a) bestehende Wirkstofformulierung auf. Dies kann z.B. durch Füllen von noch geöffneten Sachets erfolgen, die man vollständig, z.B. durch Verschweissen, schliesst. Die verschlossenen Sachets kann man anschliessend in Steckkapseln abfüllen.

Der Film oder die Folie, die nach Eindampfen einer wässrigen Lösung von Polyvinylalkohol, insbesondere Polyvinylalkohol mit einem Hydrolysegrad höher als 97 %, und Polyäthylenglycol-Fettsäureester, z.B. Polyäthylenglycol-400-stearat oder Polyäthylenglycol-2000-stearat, gegebenenfalls unter Zusatz von Weichmachern wie Glycerin, erhältlich ist, ist neu und ebenfalls Gegenstand der vorliegenden Erfindung. Diese zeichnet sich durch grosse Dehnfähigkeit aus. Besonders vorteilhafte Eigenschaften hat ein filmartiger Rückstand, der nach Eindampfen einer wässrigen Lösung enthaltend ca. 40-60 % Polyvinylalkohol, 20-40 % Polyäthylenglycolstearat und 0-30 % Glycerin erhältlich ist. Dieser Film zeichnet sich durch besonders gute Dehnfähigkeit aus.

Die vorliegenden Beispiele illustrieren die Erfindung ohne sie zu beschränken. Temperaturen sind in Graden Celsius angegeben.

### Beispiel 1:

a) 87,8 g Wasser, 2,4 g Glycerin und 9,8 g Polyvinylalkohol (Mowiol® 28-99, Hoechst) werden miteinander vermischt, gerührt und auf 95° erwärmt. Nach Abkühlen auf Raumtemperatur wird die Lösung auf eine Glasplatte gegossen, wobei eine Schicht von ca. 1 mm Stärke entsteht. Man lässt diese Schicht an der Luft trocknen, erwärmt den filmartigen Rückstand auf 100° und lässt diesen über Nacht auf Raumtemperatur abkühlen. Man erhält eine weiche, biegsame 100 µm starke Filmschicht.
   Aus dieser Filmschicht schneidet man rechteckige Streifen von ca. 3 cm Breite und 5 cm Länge aus, faltet einmal und verschweisst die Längsseiten miteinander, wobei man ein an einer Seite offenes Sachet von ca. 2 cm Innenbreite und 2,5 cm Länge erhält. Man füllt dieses Sachet mit einer Mischung bestehend aus 300 mg Natriumhydrogencarbonat und 129 mg Polyäthylenglycol-400-monostearat (PEG-400-Stearat) und verschweisst die noch offene Seite, so dass man ein verschlossenes Sachet mit einer Abgabefläche von ca. 8 cm² erhält.
b) Man gibt das Sachet bei 37° in eine wässrige Kochsalz-/Salzsäurelösung (2,0 g NaCl und 2,92 g HCl 37 % ad 1 Liter Wasser), wobei sich das ursprüngliche Volumen von ca. 0,5 ml nach 30 Minuten auf 1,5 ml und nach 8 Stunden auf 4,5 ml ausdehnt, um anschliessend nach ca. 24 Stunden auf ca. 2,9 ml abzufallen.

### Beispiel 2:

Analog dem in Beispiel 1 beschriebenen Verfahren stellt man eine Filmschicht aus 48 % Polyvinylalkohol (MOWIOL 28-99), 32 % PEG-400-Stearat und 20 % Glycerin mit einer Stärke von ca. 140 µm her, verschweisst diese zu offenen Sachets, füllt die offenen Sachets mit 300 mg Natriumhydrogencarbonat und verschweisst sie zu geschlossenen Sachets. Beim Versetzen mit wässriger Kochsalz-/Salzsäurelösung beobachtet man eine Volumenexpansion von ca. 0,5 ml auf 5,5 ml nach 30 Minuten, auf 7,8 ml nach 1 Stunde und auf 8,5 ml nach 3 Stunden. Das Volumen fällt auf 3,3 ml nach 6 Stunden und auf 1,9 ml nach 24 Stunden.

### Beispiel 3:

Analog dem in Beispiel 1 beschriebenen Verfahren stellt man eine Filmschicht aus 80 % Polyvinylalkohol (MOWIOL 28-99) und 20 % Glycerin mit einer Stärke von ca. 100 µm her, verschweisst diese zu offenen quadratischen Sachets von ca. 2 cm Seitenlänge, füllt die offenen Sachets mit 150 mg Natriumhydrogencarbonat und 150 mg kalt-wasserlöslichem Polyvinylalkohol (MOWIOL 4-88) und verschweisst sie zu geschlossenen Sachets. Beim Versetzen mit wässriger Kochsalz-/ Salzsäurelösung mit der in Beispiel 1 b) angegebenen Zusammensetzung beobachtet man eine Volumenexpansion des Sachets von ca. 0,7 ml auf 4,2 ml nach 30 Minuten und auf ca. 5,8 ml nach 2 Stunden. Das Volumen fällt auf 2,8 ml nach 6 Stunden ab.

### Beispiel 4:

Analog dem in Beispiel 1 beschriebenen Verfahren stellt man eine Filmschicht mit der in Beispiel 3 angegebenen Zusammensetzung her, verschweisst diese zu offenen quadratischen Sachets von ca. 2 cm Seitenlänge, füllt diese mit 30 mg Natriumhydrogencarbonat und 270 mg Natriumcarbonat und verschliesst sie zu geschlossenen Sachets. Beim Versetzen mit wässriger Kochsalz-/Salzsäurelösung mit der in Beispiel 1 b) angegebenen Zusammensetzung beobachtet man eine Volumenexpansion von ca. 0,6 ml auf 3,0 ml nach 2 Stunden und 4,2 ml nach 4 Stunden. Nach 24 Stunden hatte das Sachet ein Volumen von ca. 3,4 ml.

### Beispiel 5:

Analog dem in Beispiel 1 beschriebenen Verfahren stellt man eine Filmschicht aus 80 % Polyvinylalkohol (MOWIOL 28-99) und 20 % Glycerin mit einer Stärke von ca. 100 µm her, verschweisst diese zu offenen quadratischen Sachets von ca. 2 cm Seitenlänge, füllt diese mit 100 mg Natriumhydrogencarbonat und einem kleineren Sachet von einer Seitenlänge von 1,4 cm mit ca. 200 mg Natriumhydrogencarbonat und verschliesst sie zu einem geschlossenem Sachet.

Beim Versetzen mit wässriger Kochsalz-/Salzsäurelösung mit der in Beispiel 1 b) angegebenen Zusammensetzung beobachtet man eine Volumenexpansion von 0,7 ml auf folgende Werte:

| | | | | | | | | | | | |
|---|---|---|---|---|---|---|---|---|---|---|---|
| t [Std.] | 0 | 0,5 | 1,0 | 2,0 | 3,0 | 4,0 | 5,0 | 6,0 | 8,0 | 9,0 | 24,0 |
| Vol [ml] | 0,7 | 2,7 | 3,6 | 3,5 | 3,0 | 3,9 | 3,9 | 3,9 | 3,5 | 3,3 | 2,7 |

### Beispiel 6:

a) Analog dem in Beispiel 1 beschriebenen Verfahren stellt man eine Filmschicht aus 64 % Polyvinylalkohol (MOWIOL 28-99) 16 % PEG-400Stearat und 20 % Glycerin mit einer Stärke von ca. 100 µm her, verschweisst diese zu offenen quadratischen Sachets von ca. 2 cm Seitenlänge an der Innennaht, füllt diese mit 75 mg Baclofen (Lioresal®: Ciba-Geigy) und 300 mg Natriumhydrogencarbonat.
Beim Versetzen mit wässriger Kochsalz-/Salzsäurelösung mit der in Beispiel 1 b) angegebenen Zusammensetzungen beobachtet man eine Volumenexpansion von 0,6 ml auf folgende Werte:

| | | | | | | | | | | |
|---|---|---|---|---|---|---|---|---|---|---|
| t [Std.] | 0 | 0,67 | 1,0 | 2,0 | 3,0 | 4,0 | 6,0 | 7,0 | 8,0 | 24,0 |
| Vol [ml] | 0,6 | 4,5 | 4,2 | 4,7 | 5,9 | 5,0 | 3,5 | 2,8 | 3,5 | 3,2 |

b) Ein analoges Sachet mit 110 mg Baclofen gibt bei Versetzen mit 800 ml wässriger Kochsalz-/Salzsäurelösung mit der in Beispiel 1 b) angegebenen Zusammensetzung folgende Wirkstoffmenge ab:

| | | | | | | | | | |
|---|---|---|---|---|---|---|---|---|---|
| t [Std.] | 0 | 1,0 | 2,0 | 3,0 | 4,0 | 6,0 | 7,0 | 8,0 | 24,0 |
| Menge mg | 0 | 4,7 | 7,3 | 12,3 | 20,0 | 63,6 | 71,3 | 77,0 | 96,5 |

### Beispiel 7:

Analog dem in Beispiel 1 beschriebenen Verfahren stellt man eine Filmschicht aus 64 % Polyvinylalkohol (MOWIOL 28-99), 16 % PEG-400-Stearat und 20 % Glycerin mit einer Stärke von ca. 100 µm her, verschweisst diese zu offenen achteckigen Sachets von ca. 2,25 cm Durchmesser, füllt diese mit 100 mg Natriumhydrogencarbonat, 25 mg Baclofen sowie einem kleineren achteckigen Sachet mit einem Durchmesser von ca. 1,6 cm gefüllt mit 200 mg Natriumhydrogencarbonat, 86 mg PEG-400-Stearat und 50 mg Baclofen.

Beim Versetzen mit wässriger Kochsalz-/Salzsäurelösung mit der in Beispiel 1 b) angegebenen Zusammensetzung beobachtet man eine Volumenexpansion von 0,7 ml auf höhere Werte sowie folgende Abgabemengen des Wirkstoffs:

| | | | | | | | | | |
|---|---|---|---|---|---|---|---|---|---|
| t [Std.] | 0 | 1,0 | 2,0 | 3,0 | 4,0 | 6,0 | 7,0 | 8,0 | 24,5 |
| Vol [ml] | 0,7 | 2,0 | 1,8 | 2,1 | 2,9 | 2,7 | 2,8 | 2,8 | 2,0 |
| Menge mg | 0 | 1,8 | 2,6 | 3,6 | 5,2 | 7,9 | 9,2 | 10,8 | 19,5 |

### Beispiel 8:

Analog dem in Beispiel 1 beschriebenen Verfahren stellt man eine Filmschicht aus 80 % (w/w) Polyvinylalkohol (MOWIOL 28-99) und 20 % Glycerin mit einer Stärke von ca. 100 µm her und verschweisst diese zu offenen rechteckigen Sachets mit ca. 25 mm Seitenlänge. Man füllt das Sachet nacheinander mit 300 mg Natriumhydrogencarbonat, 300 mg wasserfreier Citronensäure und 50 mg Metoprolol ohne die Komponenten miteinander zu vermischen. Man evakuiert das Sachet, verschweisst es an der noch offenen Naht und erwärmt es dreissig Minuten auf 90°C. Beim Versetzen mit wässriger Kochsalz-/Salzsäurelösung mit der in Beispiel 1 angegebenen Zusammensetzung beobachtet man dreissig Minuten lang eine anfängliche Volumenexpansion auf 14 ml.

## Patentansprüche (Patentansprüche für folgende(n) Vertragsstaat(en): AT, BE, CH, DE, FR, GB, IT, LI, LU, NL, SE)

1. Umhüllte Retardform gekennzeichnet durch
a) eine bei Kontakt mit Magensaft expandierende Komponente bestehend aus einem Kohlendioxid-abgabefähigen Stoff und einem pharmazeutischen Wirkstoff,
b) eine im Magen expansionsfähige und für Magensaft permeable, die Komponente a) umgebende Umhüllung aus Polyvinylalkohol in Form eines Sachets, gegebenenfalls vermischt mit Weichmachern, und gegebenenfalls
c) eine die Komponente a) und die Membran b) umgebende Umhüllung, welche durch Einwirken von Magensaft nach der Einnahme zerfällt.

2. Umhüllte Retardform gemäss Anspruch 1, dadurch gekennzeichnet, dass der Kohlendioxid-abgabefähige Stoff Natriumhydrogencarbonat ist.

3. Umhüllte Retardform gemäss Anspruch 1, dadurch gekennzeichnet, dass der pharmazeutische Wirkstoff Baclofen ist.

4. Umhüllte Retardform gemäss Anspruch 1, dadurch gekennzeichnet, dass die Umhüllung c) kapselförmig ist.

5. Verfahren zur Herstellung der umhüllten Retardform gemäss Anspruch 1, dadurch gekennzeichnet, dass man die Komponente a) aus dem Kohlendioxid-abgabefähigen Stoff und dem pharmazeutischen Wirkstoff herstellt, diese mit der Umhüllung b) umgibt and die aus den Komponenten a) und b) bestehende Formulierungsmasse gegebenenfalls mit der zusätzlichen Umhüllung c) versieht

## Patentansprüche (Patentansprüche für folgende(n) Vertragsstaat(en): ES, GR)

1. Verfahren zur Herstellung einer umhüllten Retardform enthaltend
a) eine bei Kontakt mit Magensaft expandierende Komponente bestehend aus einem Kohlendioxid-abgabefähigen Stoff und einem pharmazeutischen Wirkstoff,
b) eine im Magen expansionsfähige und für Magensaft permeable, die Komponente a) umgebende Umhüllung aus Polyvinylalkohol in Form eines Sachets, gegebenenfalls vermischt mit Weichmachern, und gegebenenfalls
c) eine die Komponente a) und die Membran b) umgebende Umhüllung, welche durch Einwirken von Magensaft nach der Einnahme zerfällt, dadurch gekennzeichnet, dass man die Komponente a) aus dem Kohlendioxid-abgabefähigen Stoff und dem pharmazeutischen Wirkstoff herstellt, diese mit der Umhüllung b) umgibt and die aus den Komponenten a) und b) bestehende Formulierungsmasse gegebenenfalls mit der zusätzlichen Umhüllung c) versieht.

2. Verfahren zur Herstellung einer umhüllten Retardform gemäss Anspruch 1, dadurch gekennzeichnet, dass der Kohlendioxid-abgabefähige Stoff Natriumhydrogencarbonat ist.

3. Verfahren zur Herstellung einer umhüllten Retardform gemäss Anspruch 1, dadurch gekennzeichnet, dass der pharmazeutische Wirkstoff Baclofen ist.

4. Verfahren zur Herstellung einer umhüllten Retardform gemäss Anspruch 1, dadurch gekennzeichnet, dass die Umhüllung c) kapselförmig ist.

## Claims (Claims for the following Contracting State(s): AT, BE, CH, DE, FR, GB, IT, LI, LU, NL, SE)

1. A covered retard form, which comprises
a) a component that expands on contact with gastric juice, consisting of a substance capable of yielding carbon dioxide, and a pharmaceutical active ingredient,
b) a polyvinyl alcohol covering, in the form of a sachet, which surrounds component a) and which is expansible in the stomach and is permeable to gastric juice, optionally mixed with plasticisers, and optionally
c) a covering, surrounding component a) and membrane b), which disintegrates after ingestion under the action of gastric juice.

2. A covered retard form according to claim 1, wherein the substance capable of yielding carbon dioxide is sodium hydrogen carbonate.

3. A covered retard form according to claim 1, wherein the pharmaceutical active ingredient is baclofen.

4. A covered retard form according to claim 1, wherein the covering c) is in capsule form.

5. A process for the manufacture of a covered retard form according to claim 1, which comprises preparing component a) from the substance capable of yielding carbon dioxide and the pharmaceutical active ingredient, surrounding that component a) with covering b) and optionally providing the formulation composition consisting of components a) and b) with the additional covering c).

## Claims (Claims for the following Contracting State(s): ES, GR)

1. A process for the manufacture of a covered retard form, which comprises
a) a component that expands on contact with gastric juice, consisting of a substance capable of yielding carbon dioxide, and a pharmaceutical active ingredient,
b) a polyvinyl alcohol covering, in the form of a sachet, which surrounds component a) and which is expansible in the stomach and is permeable to gastric juice, optionally mixed with plasticisers, and optionally
c) a covering, surrounding component a) and membrane b), which disintegrates after ingestion under the action of gastric juice, which process comprises preparing component a) from the substance capable of yielding carbon dioxide and the pharmaceutical active ingredient, surrounding that component a) with covering b) and optionally providing the formulation composition consisting of components a) and b) with the additional covering c).

2. A process for the manufacture of a covered retard form according to claim 1, wherein the substance capable of yielding carbon dioxide is sodium hydrogen carbonate.

3. A process for the manufacture of a covered retard form according to claim 1, wherein the pharmaceutical active ingredient is baclofen.

4. A process for the manufacture of a covered retard form according to claim 1, wherein the covering c) is in capsule form.

## Revendications (Revendications pour l'(les) Etat(s) contractant(s) suivant(s): AT, BE, CH, DE, FR, GB, IT, LI, LU, NL, SE)

1. Forme retard enrobée caractérisée par
a) un composant se dilatant au contact avec le suc gastrique, constitué d'un corps délivrant du dioxyde de carbone et d'une substance active pharmaceutique,
b) une enveloppe entourant le composant a) dilatable dans l'estomac et perméable au suc gastrique, constituée d'alcool polyvinylique sous la forme d'un sachet, éventuellement mélangée avec des plastifiants et le cas échéant
c) une enveloppe entourant le composant a) et la membrane b), qui se décompose après absorption par l'action du suc gastrique.

2. Forme retard enrobée selon la revendication 1, caractérisée en ce que la matière délivrant du dioxyde de sodium est du carbonate acide de sodium.

3. Forme retard enrobée selon la revendication 1, caractérisée en ce que la substance active pharmaceutique est le Baclofen.

4. Forme retard enveloppée selon la revendication 1, caractérisée en ce que l'enveloppe c) est en forme de capsule.

5. Procédé de préparation de la forme retard enrobée selon la revendication 1, caractérisée en ce qu'on prépare le composant a) à partir de la matière délivrant le dioxyde de carbone et de la substance active pharmaceutique, en ce qu'on l'entoure avec l'enrobage b) et en ce qu'on munit la masse de formulation constituée des composants a) et b), le cas échéant, d'une enveloppe supplémentaire c).

## Revendications (Revendications pour l'(les) Etat(s) contractant(s) suivant(s): ES, GR)

1. Procédé de préparation d'une forme retard enrobée contenant
a) un composant se dilatant au contact avec le suc gastrique, constitué d'un corps délivrant du dioxyde de carbone et d'une substance active pharmaceutique,
b) une enveloppe entourant le composant a) dilatable dans l'estomac et perméable au suc gastrique, constituée d'alcool polyvinylique sous la forme d'un sachet, éventuellement mélangée avec des plastifiants et le cas échéant
c) une enveloppe entourant le composant a) et la membrane b), qui se décompose après absorption par l'action du suc gastrique, caractérisée en ce que l'on prépare le composant a) à partir de la matière délivrant le dioxyde de carbone et de la substance active pharmaceutique, en ce qu'on l'entoure de l'enveloppe b) et en ce qu'on munit le cas échéant la masse de formulation constituée des composants a) et b) de l'enveloppe supplémentaire c).

2. Procédé de préparation d'une forme retard enrobée selon la revendication 1, caractérisé en ce que la matière délivrant le dioxyde de carbone est le carbonate acide de sodium.

3. Procédé de préparation d'une forme retard enrobée selon la revendication 1, caractérisé en ce que la substance active pharmaceutique est le Baclofen.

4. Procédé de préparation d'une forme retard enrobée selon la revendication 1, caractérisé en ce que l'enveloppe c) est en forme de capsule.
